# EUROPEAN PATENT SPECIFICATION

(11) **EP 3 946 258 B1**
(45) Date of publication and mention of the grant of the patent: **21.10.2026**
(21) Application number: 20718723.8
(22) Date of filing: 30.03.2020
(51) Int. Cl.: A61K 9/00, A61K 9/51, A61K 9/70, A61K 9/50, A61K 31/513, A61P 17/00, A61P 35/00, A61K 31/60, A61K 36/76

(54) **FLUOROURACIL-CONTAINING FORMULATIONS**
FLUORURACILHALTIGE FORMULIERUNGEN
FORMULATIONS CONTENANT DU FLUOROURACILE

(30) Priority: 28.03.2019 GB 201904338
(43) Date of publication of application: 09.02.2022
(73) Proprietor: SiSaf Ltd, Guildford GU2 7YD (GB)
(72) Inventor: SAFFIE-SIEBERT, Roghieh Suzanne, Guildford Surrey GU27RE (GB); SUTERA, Flavia Maria, Guildford Surrey GU27RE (GB); TORABI-POUR, Nasrollah, Guildford Surrey GU27RE (GB)
(74) Representative: Abel & Imray LLP
(86) International application number: PCT/GB2020/050850
(87) International publication number: WO 2020/193996

(56) References cited:
- WO-A1-2011/012867
- WO-A1-2017/120537
- US-A1- 2009 053 268
- US-B1- 8 992 984
- US-B2- 6 670 335

## Description

### FIELD OF THE INVENTION

The invention relates to an improved topical formulation containing fluorouracil and to its uses.

### BACKGROUND TO THE INVENTION

Fluorouracil (International Non-proprietary Name) is the chemical 5-fluoro-2,4 (1H,3H)-pyrimidinedione. It is useful as an anti-cancer drug and has been used for systemic treatment of various cancers, including those of the breast, bladder and pancreas. It is also used topically to treat superficial basal cell carcinomas, actinic keratoses, solar keratoses and various forms of scarring and also severe acne. Topical formulations containing fluorouracil are currently available, but, whilst effective, they may cause side effects, the principal side effect being irritation of the skin and related pain, ulceration, erythema etc. There exists a need for improved formulations which deliver an effective transdermal dose of fluorouracil whilst minimizing these side effects.

US 6,670,335 discloses the use of formulations consisting of oil in water emulsions wherein fluorouracil is present in porous micro-particles referred to as "micro sponges" and also within the emulsion, presumably in the aqueous phase of the emulsion due to its hydrophilicity.

There remains a continuing need for improved delivery systems for topically applied fluorouracil.

The present invention relates to improved formulations using silicon nanoparticles having better properties than the micro-particles of US 6,670,335 and wherein the fluorouracil is substantially entirely associated with silicon nanoparticles having superior properties. Those nanoparticles are in turn encapsulated in a lipid matrix comprising one or more waxy fatty acid esters, which is substantially free of fluorouracil and which may be processed into a powder suitable for various topical formulations which give good bioavailability following application and reduced side effects.

### Silicon Nanoparticles

A number of ways of delivering pharmaceutically active ingredients in a controlled or slow-release manner have been developed. However, little attention has previously been paid to the fate of the carrier material once it has performed its function of delivering and releasing the active ingredient. This invention uses a delivery system in which a silicon-based carrier material is converted to a beneficial substance following administration.

To enable active ingredients to be delivered topically, considerable research has been focused on development of strategies for temporarily disrupting the stratum corneum barrier in a controllable fashion, so that drugs can permeate in sufficient and predictable quantities, thus attaining therapeutic levels. While some techniques such as iontophoresis and ultrasound have been explored as skin absorption enhancers, most effort has centred on identifying non-toxic chemical penetration enhancers that could reversibly interact with the stratum corneum in order to allow greater amounts of drug to permeate the skin. Early attempts to disrupt the barrier used simple solvents or solvent mixtures, surface-active agents and fatty acids. These materials, although capable of increasing the penetration of many chemicals across the skin, were often associated with undesirable side effects linked to their ability to extract or interact with skin components, thereby eliciting an irritation response.

Silicon is an essential trace element for plants and animals. Silicon has a structural role as a constituent of the protein-glycosaminoglycan complexes found in the connective tissue's matrix of mammals, as well as a metabolic role in growth and osteogenesis (the presence of silicon promotes the process of mineralisation of the bone). Thus, silicon is essential for the normal development of bones and connective tissue. Silicon is also known to play an important role in skin health, acting as a collagen and elastin promoter and being involved in antioxidative processes in the body. It is implicated in the production of glycosaminoglycans and silicon-dependant enzymes increase the benefits of natural tissue building processes.

For medical applications, silicon can be produced as micro- or nanoparticles, which facilitates its administration via a variety of routes such as topical, oral intake, injection or implant. Biodegradable silicon-based particles have also been used for drug targeting. However, the bioavailability of silicon is often limited by poor solubility and organic silicon-containing materials tend to exhibit unacceptably high toxicity, limiting their use in cosmetic, skin care and pharmaceutical applications.

Porous silicon was first discovered by accident in 1956 by Arthur Ulhir Jr. and Ingeborg at the Bell laboratories in US. Fabrication of porous silicon may range from its initial formation through use of stain-etching or an anodization cell using single or polycrystal silicon immersed in hydrofluoric acid (HF) solution. Creating pores in the silicon allows for both the degradation of the material and the loading of active compounds into the silicon pores. The use of porous silicon as a carrier for other active compounds has been described (Saffie-Siebert R et al., Drug Discovery World 2005; 6: 71-6; Saffie-Siebert, R et al., Pharmaceutical Technology Europe, 17(4), 21-28 (2005); Luo, D., Saltzman, W. M., Gene Therapy (2006) 13, 585-586; Ahola, M., Kortesuo, P., Kangasniemi, I., Kiesvaara, J., Yli-Urpo, A., Int. J. Pharm. 195 (2000) 219 227. Ahola. M., Säilynoja, E.S., Raitavuo, M.H., Vaahtio, M.H., Salonen, J.I., Yli-Urpo, A.U.O., Biomat. (2001), 15, 2163-2170; Lu, J. , Liong, M., Zink, J., Tamanoi, F, Small. 2007, 3: 1341-1346). However, the importance of the degraded product of such carrier systems must also receive attention. In particular, a silicon-containing carrier system preferably degrades to form the beneficial and bioactive form of silicon, orthosilicic acid, without polymerisation.

The dissolution products of silicon within an aqueous environment are silicic acids. Silicic acid is a general name for a family of chemical compounds of the elements silicon, hydrogen, and oxygen, with the general formula [SiOₓ(OH)₄₋₂ₓ]ₙ. Some simple silicic acids have been identified in very dilute aqueous solutions, such as metasilicic acid (H₂SiO₃), orthosilicic acid (H₄SiO₄, pKₐ₁ = 9.84, pKₐ₂ = 13.2 at 25 °C), disilicic acid (H₂Si₂O₅), and pyrosilicic acid (H₆Si₂O₇); and further polymerised silicic acids (PolySA), with silica (SiO₂) representing the end point of complete polymerisation. The monomeric form of sicilic acid, orthosilicic acid (OSA), alternatively known as monosilicic acid, and silica represent opposite sides of the silicon-based reactions with silica representing the energetically favourable form. Concentration and pH determine the direction of reaction and the equilibrium between monomers, polymers and silica:

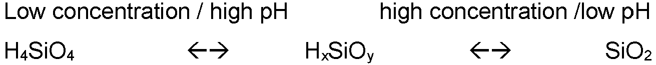

Silicic acids can be considered as buffer molecules. Orthosilicic acid (OSA) is a very weak acid, weaker than, for instance, carbonic acid. It dissociates with a pK₁ of 9.84 at 25 °C according to:

H₃SiO₄⁻ + H₃O⁺ ↔ H₄SiO₄ + H₂O

H₄SiO₄ + OH- ↔ H₃SiO₄- + H₂O

Silicic acid has a pKa around 9.8, and thus represents a mixture of ionised and undissociated acids in solution. The ionised species (H₃SiO₄⁻) acts as a proton scavenger, removing protons from solution and thus raising the pH of the solution. Whereas the undissociated species can donate a proton to neutralise the hydroxide ions, thus raising the pH of the solution. In this manner, the silicic acid buffers the solution. It is worth noting that this buffering capacity occurs quickly at low Si concentrations. At high Si concentrations, low pH promotes silicic acid to undergo condensation reactions to produce dimers (H₆Si₂O₇) or higher structures, and water. These dimers and higher structures (SiO ₓOH_{y}) can dissociate back to monomers or lower structures by reacting with hydroxide ions present in solution, thereby lowering the pH. Likewise, these polymerised acids also dissociate at high pH, by neutralising the hydroxide. Thus, these polysilicic acids can also act as a buffer, albeit the reactions are considerably slower.

Silica [SiO2] represents the end point of complete polymerisation of OSA, which reduces its solubility and hence bioavailability, biodegradability, and safety.

H₄SiO₄ → 2 H₂O + SiO₂

Due to the enthalpy of the dimerization reaction, and the subsequent polymerisation reactions, at ambient temperatures and under biological pH, polymerisation generally proceeds *via:*

H₄SiO₄ + H₄SiO₄ → H₂O + H₆Si₂O₇

[SiₙOₘ]-OH + H₄SiO₄ → [Siₙ₊₁Oₘ₊₂]-OH + 2 H₂O

This is a reversible process, therefore the back reaction from silica to OSA is theoretically possible; nevertheless, it is thermodynamically unfavourable in physiological conditions, as it requires pH values above 13 and high temperatures.

The reaction of OSA with itself to form silica can be limited by reducing its concentration to the point where the probability of two OSA molecules meeting in solution is as likely as a silicic acid dimer meeting an OH⁻ ion in solution and dissociating. The limiting concentration of a pure solution containing only silicic acid is around 10⁻⁴ Mol.L⁻¹ (Studies of the kinetics of the precipitation of uniform silica particles through the hydrolysis and condensation of silicon alkoxides, Journal of Colloid and Interface Science, Volume 142, Issue 1, 1 March 1991, Pages 1-18 G.H Bogush and C.F Zukoski IV) and above this concentration one cannot identify pure OSA as other PolySA species are formed. At higher concentrations, however, OSA can be prevented from undergoing polymerisation through the addition of other chemical species.

Kinetics of dissolution:

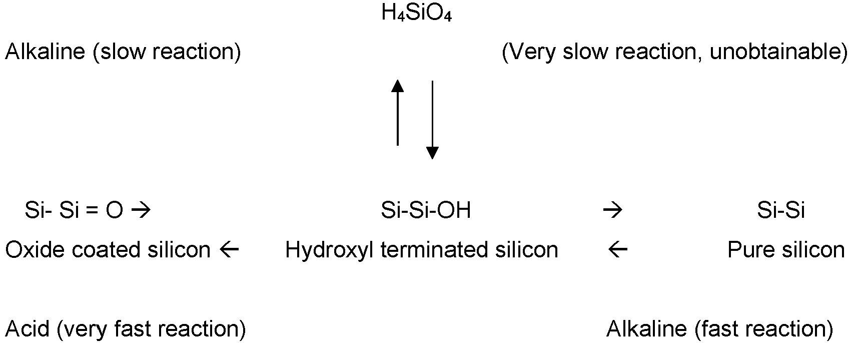

The kinetics of dissolution, ignoring surface area, are dependent on the pH and the availability of reactive species. The main reactive species in the dissolution process is water in its protonated and deprotonated forms (for kinetic data on the rates of reaction in both directions, see Brinker sol-gel science and technology). The addition of other molecules however, can give rise to side reactions, which can greatly shift the equilibrium to silicic acid or silicon oxide (glass), subject to the pKa value of those other molecules.

The control of dissolution through adjustment of pH is possible for storage applications, however the pH *in vivo* is tightly controlled by the body. Thus adjustment of dissolution rates through particle size and surface chemistry must be tailored prior to *in vivo* use. Increasing the rate of dissolution of pure, protonated or hydroxylated silicon is preferable. If slow dissolution of the silicon particles is desired, an oxide layer of suitable thickness will produce a lag in the dissolution profile whilst the oxide layer slowly dissolves. The thickness of this oxide layer will determine the length of the lag period before any water has access to the silicon core.

Care may need to be taken with the manipulation of the silicon surface as binding of drug molecule will be highly dependent on the surface energy.

The growth of a surface oxide will increase contact angle, favouring the binding of hydrophobic molecules and decreases the binding of polar molecules. Whilst hydroxylation of the surface will reduce contact angle between the silicon surface and the inbound drug molecule, favouring the binding of hydrophilic molecules such us fluorouracil.

OSA is a very weak acid which is unstable stable at pH levels lower than 9.5 and quickly precipitates out of solution, or forms sols or gels which are not very bioavailable for the human body. It is therefore very difficult to prepare highly concentrated (> 0.5% silicon) solutions of orthosilicic acid and oligomers. Furthermore, the type of silicic acid produced by a formulation is largely determined by the concentration of silicic acids, silicon compounds, and the pH of the media in which this dissolution occurs. In order to obtain OSA *in vivo,* the silicic acid concentration must be tightly controlled.

WO 2011/012867 proposes the use of stabilised silicon-based materials as delivery agents for beneficial compounds. The stabilisation is carried out in order to control the degradation of elemental silicon to biologically active orthosilicic acid with low levels of polysilicic acid (polySA) production, thus providing better product safety.

The present invention is based on the realisation that silicon nanoparticles which are stabilised with a stabilising agent in accordance with the method of WO 2011/012867 not only provide the advantages attributable to the invention of WO 2011/012867 - namely improved degradation to bioavailable OSA, but that those stabilised silicon nanoparticles are especially good at binding and delivering fluorouracil in such a way that sufficient fluorouracil can be loaded onto the stabilised silicon nanoparticle and released where needed, by processes including silicon degradation such that stabilised silicon nanoparticles can be encapsulated in a waxy lipid to produce a powder comprising solid particles wherein the waxy lipid and any surrounding medium into which it is formulated for topical administration is substantially free of fluorouracil. This is in contrast to the formulations of US 6,670,335, where fluorouracil is present in significant amounts not associated with particles. The present invention allows a therapeutically effective dose to be administered whilst mitigating the side effects of skin surface burning and irritation caused by dose dumping of fluorouracil on the skin following initial application.

Advantages of using silicon nanoparticles over the micro-particles of the prior art are that the silicon material itself is biocompatible, biodegradable and a highly tuneable system which can be made in an optionally highly porous nanoparticle size of from 20 to 400 nm which is ideal for skin delivery because it is too small to block pilosebaceous ostra or sweat ducts (pores), but its small size allows the particles to actively penetrate to the bottom of the hair follicles rather than merely act as a surface drug reservoir.

The use of silicon nanoparticles is especially suitable for use in compositions comprising fluorouracil because it allows the hydrophilic fluorouracil to be formulated into hydrophobic waxy powder particles, also known as waxy microspheres, which are otherwise only suitable for hydrophobic compounds.

### SUMMARY OF THE INVENTION

According to a first aspect, the invention provides pharmaceutically compatible nanoparticles comprising at least 50% by weight hydrolysable silicon surface coated with a phospholipid, wherein the coated nanoparticles are associated with fluorouracil.

According to a second aspect, the invention provides a pharmaceutically compatible powder comprising solid particles of one or more waxy fatty acid esters into which are encapsulated pharmaceutically compatible nanoparticles according to the first aspect of the invention, wherein more than 90% by weight of the fluorouracil of the composition is associated with the optionally coated nanoparticles. Preferably, the powder comprises salicylates, for example the powder may comprise willow bark extract.

According to a third aspect, the invention provides a pharmaceutically compatible cream or gel, suitable for topical application to the skin or other body surface, comprising a cream or gel base into which a pharmaceutically compatible powder according to the second aspect of the invention is suspended.

According to a fourth aspect, the invention provides an adhesive patch comprising a backing layer and an adhesive film wherein the adhesive film comprises a pharmaceutically compatible powder according to the second aspect of the invention or a cream or gel according to the third aspect of the invention.

According to a fifth aspect, the invention provides pharmaceutically compatible nanoparticles according to the first aspect of the invention, a pharmaceutically compatible powder according to the second aspect of the invention, a pharmaceutically compatible cream or gel according to the third aspect of the invention or an adhesive patch according to the fourth aspect of the invention for use as a medicament.

According to a sixth aspect, the invention provides pharmaceutically compatible nanoparticles according to the first aspect of the invention, a pharmaceutically compatible powder according to the second aspect of the invention, a pharmaceutically compatible cream or gel according to the third aspect of the invention or an adhesive patch according to the fourth aspect of the invention for use as a medicament for treating superficial basal cell carcinoma or actinic keratoses, solar keratoses, scarring or acne.

### DETAILED DESCRIPTION OF THE INVENTION

### Definitions

According to the present disclosure, a derivative of a compound may be a compound having substantially the same structure, but having one or more substitutions. For example, one or more chemical groups may be added, deleted, or substituted for another group. In certain preferred embodiments, the derivative retains at least part of a pharmaceutical or cosmetic activity of the compound from which it is derived, for example at least 90%, 80%, 70%, 60%, 50%, 40%, 30%, 20% or 10% of an activity of the compound from which it is derived. In some embodiments, the derivative may exhibit an increased pharmaceutical or cosmetic activity compared to the compound from which it is derived.

For example, in the context of a peptide, a peptide derivative may encompass the peptide wherein one or more amino acid residues have been added, deleted or substituted for another amino acid residue. In the case of a substitution, the substitution may be a non-conservative substitution or a conservative substitution, preferably a conservative substitution.

According to a first aspect, the invention provides pharmaceutically compatible nanoparticles comprising hydrolysable at least 50% by weight silicon, surface coated with a phospholipid (for example, one or more of phosphatidylcholine, hydrogenated phosphatidylcholine, phosphatidylethanolamine, components of lecithin, particularly one or more of phosphatidylcholine, hydrogenated phosphatidylcholine) wherein the coated nanoparticles are associated with fluorouracil.

The coating of phospholipid preferably modifies the rate of hydrolysis of the silicon and/or inhibits the rate of orthosilicic acid polymerisation. Preferably, it inhibits the rate of hydrolysis of the silicon-containing material.

In one embodiment, the rate of hydrolysis of the silicon containing material is modified by the presence of the phospholipid (for example, one or more of phosphatidylcholine, hydrogenated phosphatidylcholine, phosphatidylethanolamine, components of lecithin, particularly one or more of phosphatidylcholine, hydrogenated phosphatidylcholine) such that the rate is less than 50% of the rate of hydrolysis of an identical composition without the phospholipid, preferably less than 30%, especially less than 10%.

By slowing the rate of hydrolysis to a level below that at which OSA is assimilated by the body or removed from the delivery site, for example by diffusion, it has been found that OSA polymerisation can be avoided or at least lessened, and the beneficial effects of delivery of OSA to the body can be realised.

As the monomeric silicic acid degradation product is naturally available in the human body, the use of products of the invention bears a very low risk of toxicity, which is a significant advantage over many other delivery systems. The delivery system according to the invention affords the additional advantage that the carrier decomposes to provide a bioavailable compound which is known to be beneficial. For example, OSA is known to stimulate cellular proliferation and migration in certain cell types, including fibroblasts, endothelial cells and keratinocytes.

Advantageously, the bioavailable orthosilicic acid resulting from degradation of the nanoparticles according to the invention (for example, nanoparticles coated with one or more of phosphatidylcholine, hydrogenated phosphatidylcholine, phosphatidylethanolamine, components of lecithin, particularly one or more of phosphatidylcholine, hydrogenated phosphatidylcholine) may itself be beneficial as a nutrient for skin, bones, hair, nails, connective tissue, and for the treatment or prevention of bone or joint conditions such as arthritis or osteoporosis.

It has been found that silicon nanoparticles that have been surface coated with phospholipid, especially if the phospholipid coating is in the form of one or more phospholipid bilayers, are especially suitable for associating with fluorouracil. This association is preferably brought about by attraction between opposite charges, for example it may be an electrostatic association or an ionic bond between charges of the phospholipid bilayer and/or those on the surface of the silicon nanoparticle, and charges on the fluorouracil. According to preferred embodiments relevant to all aspects of the invention, the association is promoted by the presence of amino acids and, therefore, all products of the invention (for example, nanoparticles surface coated with one or more of phosphatidylcholine, hydrogenated phosphatidylcholine, phosphatidylethanolamine, components of lecithin, particularly one or more of phosphatidylcholine, hydrogenated phosphatidylcholine) also preferably comprise amino acids, in particular arginine or a mixture of arginine and glycine.

The presence of amino acids (for example, one or more of arginine and glycine) also helps to stabilise the surface charge of the silicon nanoparticle and improve its association with the phospholipid (for example, one or more of phosphatidylcholine, hydrogenated phosphatidylcholine, phosphatidylethanolamine, components of lecithin, particularly one or more of phosphatidylcholine, hydrogenated phosphatidylcholine) and fluorouracil. Thus, the presence of one or more amino acids helps control both the release of the fluorouracil and the stability and degradation rate of the silicon over time. In its broadest sense, the term "amino acid" encompasses any artificial or naturally occurring organic compound containing an amine (-NH₂) and carboxyl (-COOH) functional group. It includes an α, β, γ and δ amino acid. It includes an amino acid in any chiral configuration. According to some embodiments, it is preferred to be a naturally occurring α amino acid. It may be a proteinogenic amino acid or a non-proteinogenic amino acid (such as carnitine, levothyroxine, hydroxyproline, ornithine or citrulline). It is especially preferred to comprise arginine, or glycine or a mixture of arginine and glycine. Preferably, the 30% of the amino acid present is arginine. Accordingly, preferred pharmaceutically compatible nanoparticles of the invention (for example, silicon nanoparticles coated with one or more of phosphatidylcholine, hydrogenated phosphatidylcholine, phosphatidylethanolamine, components of lecithin, particularly one or more of phosphatidylcholine, hydrogenated phosphatidylcholine) are such that the coated nanoparticles are associated with fluorouracil and an amino acid (preferably selected from arginine, glycine and mixtures thereof, most preferably both arginine and glycine).

The presence of willow bark extract associated with the nanoparticles of the invention (for example, nanoparticles coated with one or more of phosphatidylcholine, hydrogenated phosphatidylcholine, which are associated with fluorouracil, and which are optionally associated with one or more of arginine and glycine) also helps to improve the association of the nanoparticle with fluorouracil. Willow bark extract (extracted from the bark of *Salix nigra* and/or *Salix alba,* preferably *Salix nigra*) provides a matrix in which the fluorouracil may become entrapped, leading to an increased association of fluorouracil with the nanoparticles. This should help to ensure the controlled release of fluorouracil upon delivery of the nanoparticles to a treatment site, for example when nanoparticles of the invention are delivered topically to the surface of the skin, such as in a cream or gel. Moreover, willow bark extract typically comprises salicin, which is metabolised to form salicylic acid and is known to exhibit anti-inflammatory and antioxidant activity.

According to preferred embodiments, at least 80%, for example at least 90% of the fluorouracil by weight present in the products of all aspects of the invention is associated with the coated nanoparticles (for example, nanoparticles coated with one or more of phosphatidylcholine, hydrogenated phosphatidylcholine, which are associated with fluorouracil and which may also be associated with willow bark extract and/or one or more amino acids such as one or more of arginine and glycine).

Molecular association between fluorouracil and the phospholipid-coated silicon nanoparticle advantageously ensures that the fluorouracil becomes bio-available as the silicon nanoparticle or the coating thereof degrades. Because the rate of degradation by hydrolysis, being the principal rate of degradation, can be controlled, the rate at which fluorouracil becomes bio-available can also be controlled in order to avoid dose-dumping and/or to ensure release only when the nanoparticles have found their way to a location away from the skin surface (for example a basal location).

Nanoparticles according to all aspects of the invention (for example, nanoparticles coated with one or more of phosphatidylcholine, hydrogenated phosphatidylcholine, which are associated with fluorouracil and which may also be associated with willow bark extract and/or one or more amino acids such as one or more of arginine and glycine) are preferably porous. For example, their porosity may increase their surface area by a factor of at least 1.5, 2, 2.5, 3, 3.5 or 4 over the surface area of an equivalently sized non-porous material.

### Phospholipids

The phospholipid for use in accordance with all aspects of the invention (for example, one or more of phosphatidylcholine, hydrogenated phosphatidylcholine, phosphatidylethanolamine, components of lecithin, particularly one or more of phosphatidylcholine, hydrogenated phosphatidylcholine) is a compound that optionally modifies, for example reduces or nullifies, the rate of hydrolysis of a silicon containing material in an aqueous solution, for example in phosphate buffered saline (PBS), and/or stabilises OSA in such a solution once formed by inhibiting the rate of polymerisation of OSA thus generating an inert carrier. Accordingly, the phospholipid may, for example, be an agent that promotes the formation of OSA on hydrolysis of a silicon containing material in an aqueous solution, in particular in a commonly used aqueous buffer solutions such as tris or phosphate buffered saline, and/or which inhibits the rate of OSA polymerisation in aqueous solution following hydrolysis of the silicon-containing material for more than 24hrs.

Generally, PBS contains the following constituents: 137 mM NaCl, 2.7 mM KCI, 10 mM sodium phosphate dibasic, 2 mM potassium phosphate monobasic and a pH of 7.4. PBS is used as a model of physiological conditions at a temperature of 37 °C.

As discussed above, silicon hydrolyses to OSA in aqueous media and then subsequently polymerises into molecular entities of various chain lengths and structures, eventually forming water-insoluble silicates. The products according to the present invention optimise the biodegradation process, so that polymerisation of the OSA formed is substantially suppressed. In this way the degradation product is stabilised and its properties, particularly solubility and viscosity, are controlled in order to maximise bioavailabilty. This is achieved by chemical modification of the nanoparticle surface, the surface being coated with the phospholipid stabilising agent (for example, one or more of phosphatidylcholine, hydrogenated phosphatidylcholine) and optionally with one or more amino acids by surface association (for example, one or more of arginine and glycine). Optionally, the nanoparticle is also associated with willow bark extract.

In the absence of a phospholipid coating, polymerisation proceeds rapidly with OSA concentrations of over 10⁻⁴ M, which corresponds to 9.6 mg/L or 0.48 mg/50 mL. In one embodiment the phospholipid coating is capable of stabilising a solution of OSA at concentrations higher than 10⁻⁴ M mg/L, for example, a concentration of 0.5 mg/50 mL or more, especially concentration of 0.80 mg/50 mL or more. Advantageously, the phospholipid coating is capable of stabilising OSA solutions of 0.90 mg/50 mL or more, for example 0.95 mg/50 mL or more, especially 1.0 mg/50 mL or more.

In one embodiment, the products of the first aspect of the invention (which optionally comprise willow bark extract and/or one or more amino acids such as one or more of arginine and glycine) comprise at least 5% by weight phospholipid (for example, one or more of phosphatidylcholine, hydrogenated phosphatidylcholine, phosphatidylethanolamine, components of lecithin, particularly one or more of phosphatidylcholine, hydrogenated phosphatidylcholine) for example at least 20 wt%, typically at least 30 wt% and especially at least 50 wt% phospholipid based on the total weight of the coated nanoparticle. In one embodiment the molar ratio of the phospholipid to silicon is at least 0.8 to 1, for example at least 1 to 1, typically at least 1.5 to 1. It has been found that a phospholipid to silicon molar ratio of at least 2 to 1 is particularly advantageous.

In one embodiment, the phospholipid (for example, one or more of phosphatidylcholine, hydrogenated phosphatidylcholine, phosphatidylethanolamine, components of lecithin, particularly one or more of phosphatidylcholine, hydrogenated phosphatidylcholine) has a number average molecular weight in the range of from 500 to 1000. Particularly suitable phospholipids are glycerophospholipids. Particularly suitable phospholipids are those in which the polar head group is linked to quaternary ammonium moieties, such as phosphatidylcholine (PC) or hydrogenated phosphatidylcholine. The type of phospholipid may be selected in dependence of the nature of the formulation with neutral or negatively charges lipid being preferred for aprotic formulation while positive charge and small CH₃ chain lipids being preferred for protic formulations. Preferably the side chain(s) is/are (an) aliphatic side chain with 15 or more carbon atoms or an ether side chain with 6 or more repeating ether units, such as a polyethylene glycol or polypropylene glycol chain.

In one embodiment, the phospholipid stabilizing agent (for example, one or more of phosphatidylcholine, hydrogenated phosphatidylcholine, phosphatidylethanolamine, components of lecithin, particularly one or more of phosphatidylcholine, hydrogenated phosphatidylcholine) is an electrostatically absorbed species that binds to the surface of the silicon by van der Waal's forces. Preferably, the stabilizing agent has a contact angle less than 45 °, more preferably less than 20 ° and ideally less than 10 ° measured by optical densitometry, wherein the contact angle of a drop of the stabilising agent on surface of silicon wafer is observed and measured. The lower the contact angle the greater the interaction between the surface and the stabilising agent. Chemical features that result in a good van der Waal's attraction include hydrogen saturated molecules, such as saturated lipids. Phospholipids have an amphiphilic character with a hydrophilic "head" and a lipophilic "tail" or "tails".

Phospholipids can spontaneously form phospholipid bilayers wherein the changed head groups face outwards and the lipidic tails face inwards. According to preferred embodiments (for example, when the nanoparticles are coated with one or more of phosphatidylcholine, hydrogenated phosphatidylcholine; such nanoparticles may optionally be associated with willow bark extract and/or one or more amino acids such as one or more of arginine and glycine) the phospholipid coating the surface of the nanoparticles of the invention is present as a phospholipid bilayer, for example a phospholipid bilayer comprising phosphatidylcholine or hydrogenated phosphatidylcholine.

Other suitable phospholipids for use in accordance with all aspects of the invention in addition to or as an alternative to phosphatidylcholine include phosphatidylethanolamine, lecithin components, phosphoinositides (for example phosphatidylinositol, phosphatidylinositol phosphate, phosphatidylinositol biphosphate and phosphatidylinositol triphosphate) and phosphosphingolipids such as ceramide phosphorylcholine, ceramide phosphorylethanolamine and ceramide phosphorylipid. For example, one or more of these phospholipids may be used when the nanoparticles are associated with willow bark extract and/or one or more amino acids such as one or more of arginine and glycine. The phospholipid used in accordance with the invention may of course be used as a mixture of phospholipids. For example, a mixture of phospholipids may be used when the nanoparticles are associated with willow bark extract and/or one or more amino acids such as one or more of arginine and glycine. The phospholipid may also be used in a mixture of phospholipids and minor non-phospholipid components - for example, other lipids or sterols such as cholesterol, which may be useful in fine tuning the properties of the phospholipid bilayer, may be included in the coating. For example, a mixture of phospholipids and minor non-phospholipid components may be used when the nanoparticles are associated with willow bark extract and/or one or more amino acids such as one or more of arginine and glycine. The phospholipid coating preferably comprises at least 60% phospholipids, for example at least 70 or 80% phospholipid. In certain embodiments the phospholipid coating comprises at least 60, 70 or 80% phosphatidylcholine or hydrogenated phosphatidylcholine (for example, when the nanoparticles are associated with willow bark extract and/or one or more amino acids such as one or more of arginine and glycine). Preferably, the coating comprises a bilayer consisting of at least 80% hydrogenated phosphatidylcholine.

Because nanoparticles of the invention may be used to produce powders of the second aspect of the invention in a process which includes the use of melted waxy fatty acid ester, the phospholipid coating (for example, a phospholipid coating comprising one or more of phosphatidylcholine, hydrogenated phosphatidylcholine, phosphatidylethanolamine, components of lecithin, particularly one or more of phosphatidylcholine, hydrogenated phosphatidylcholine) is preferably able to withstand heating, for example heating to 30 °C, 35 °C, 40 °C, 45 °C, 50 °C or 55 °C.

Preferably, the phospholipid coating is a phospholipid bilayer (for example, a phospholipid bilayer comprising one or more of phosphatidylcholine, hydrogenated phosphatidylcholine, phosphatidylethanolamine, components of lecithin, particularly one or more of phosphatidylcholine, hydrogenated phosphatidylcholine).

For example, the phospholipid coating may be a phospholipid bilayer comprising at least 80% hydrogenated phosphatidylcholine which remains substantially intact when heated to 30 °C, 35 °C, 40 °C, 45 °C, 50 °C or 55 °C for 20 minutes.

### Nanoparticles Comprising Hydrolysable Silicon

Products of the invention comprise silicon nanoparticles. The silicon nanoparticles are surface coated with a phospholipid and are associated with fluorouracil. Optionally, the silicon nanoparticles are also associated with willow bark extract and/or one or more amino acids such as one or more of arginine and glycine. The silicon nanoparticles have a nominal diameter of between 10 and 400 nm, for example 50 to 350 nm, for example 80 to 310 nm, for example 100 to 250 nm, for example 120 to 240 nm, for example 150 to 220 nm, for example about 200 nm. They are made of either pure silicon or a hydrolysable silicon-containing material. They are preferably porous. Silicon nanoparticles can be made porous by standard techniques such as contacting the particles with a hydrofluoric acid (HF)/ethanol mixture and applying a current. By varying the HF concentration and the current density and time of exposure, the density of pores and their size can be controlled and can be monitored by scanning electron micrography and/or nitrogen adsorption desorption volumetric isothermic measurement.

### Fluorouracil

Fluorouracil is electrostatically associated with the surface of the silicon nanoparticles and/or the phospholipid bilayer (for example, a phospholipid bilayer comprising one or more of phosphatidylcholine, hydrogenated phosphatidylcholine, phosphatidylethanolamine, components of lecithin, particularly one or more of phosphatidylcholine, hydrogenated phosphatidylcholine) Preferably at least 90% of the total fluorouracil in the products of the invention (for example, silicon nanoparticles coated with one or more of phosphatidylcholine, hydrogenated phosphatidylcholine, and optionally associated with willow bark extract and/or one or more amino acids such as one or more of arginine and glycine) is physically associated with or absorbed onto the surface of the silicon nanoparticles and/or the phospholipid bilayer. That is to say less than 10% of the total fluorouracil is free.

### Willow bark extract

Products of the invention (for example, silicon nanoparticles coated with one or more of phosphatidylcholine, hydrogenated phosphatidylcholine and derivatives thereof, associated with fluorouracil and optionally associated with one or more amino acid such as one or more of arginine and glycine) may comprise willow bark extract. Willow bark extract is commercially available from a number of sources. For example, willow bark extract may be obtained from Active Concepts, Srl., 9 Via Petrolo Litta, 20010 Bareggio (Milano) Italy. Willow bark extract typically comprises salicin, the structure of which is shown below:

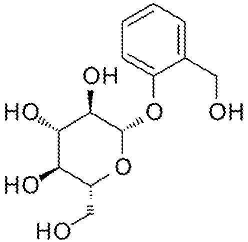

Salicin is a β-glucoside and is a derivative of salicylic acid. Salicin is typically metabolised to salicylic acid in the human body. When salicin is metabolised, its acetalic ether bridge breaks down, resulting in glucose and salicyl alcohol. Salicylic acid then results from oxidation of the alcohol group in salicyl alcohol.

Willow bark extract may be extracted from the bark of *Salix nigra* or *Salix alba,* preferably from the bark of *Salix nigra.* Willow bark extract may be provided in products of the invention as a powder, such as a powder derived from powdered willow bark. Alternatively, willow bark extract may be provided in products of the invention in solution, such as in an aqueous solution or an ethanolic solution. Liquid willow bark extract is typically colourless to light amber in colour.

Willow bark extract is known to exhibit antioxidant activity, as well as anti-inflammatory activity. Willow bark extract can therefore be used as an active ingredient in anti-aging formulations. Willow bark extract is often sold for its analgesic properties, because it typically contains from 8 to 12 wt % salicin (or, more generally, from 8 to 12 wt % salicylates). For this reason, commercially available willow bark extract is often characterized by the wt % salicin, salicylates or salicylic acid that it contains.

### Powders

According to a second aspect, the invention provides a pharmaceutically compatible powder comprising solid particles of one or more waxy fatty acid esters, into which are encapsulated pharmaceutically compatible nanoparticles according to the first aspect of the invention (for example, nanoparticles coated with one or more of phosphatidylcholine, hydrogenated phosphatidylcholine, which may also be associated with willow bark extract and/or an amino acid such as one or more of arginine and glycine) wherein more than 65% by weight of the fluorouracil of the composition is associated with the coated nanoparticles. Preferably less than 10% by weight of the fluorouracil of the composition is present in the waxy fatty acid ester portion of the composition.

The powder (for example, powder comprising solid particles of one or more waxy fatty acid esters, into which are encapsulated silicon nanoparticles coated with one or more of phosphatidylcholine, hydrogenated phosphatidylcholine, the coated silicon nanoparticles being associated with fluorouracil and, optionally, willow bark extract and/or an amino acid such as one or more of arginine and glycine) preferably comprises approximately spherical particles having their largest dimension between 30 and 550 microns. For example, at least 90% of the particles may have their largest dimension as between 50 and 500 microns (or 100 and 500 microns or 150 and 400 microns). Because the solid particles of the powder of the invention are significantly larger than the nanoparticles of the invention, each particle will typically encapsulate multiple nanoparticles of the invention.

The waxy fatty acid esters (for example, waxy fatty acid esters into which are encapsulated silicon nanoparticles coated with one or more of phosphatidylcholine, hydrogenated phosphatidylcholine, the coated silicon nanoparticles being associated with fluorouracil and, optionally, willow bark extract and/or an amino acid such as one or more of arginine and glycine) preferably have a melting point of between 25 °C and 45 °C, for example between 28 °C and 42 °C, for example between 30 °C and 40 °C. Their melting point is preferably such that they melt on skin contact. According to certain embodiments (for example, when the waxy fatty acid esters encapsulate silicon nanoparticles coated with one or more of phosphatidylcholine, hydrogenated phosphatidylcholine, the coated silicon nanoparticles being associated with fluorouracil and, optionally, willow bark extract and/or an amino acid such as one or more of arginine and glycine) the waxy fatty acid esters are esters of stearyl alcohol, although esters of other fatty alcohols may be used, particularly alcohols of saturated fatty acids, for example esters of caprylic, decanoic, lauric, myristic, palmitic and oleic alcohol. Preferably, the fatty component of the esters is heptanoic acid or caprylic acid. According to preferred embodiments (for example, when the waxy fatty acid esters encapsulate silicon nanoparticles coated with one or more of phosphatidylcholine, hydrogenated phosphatidylcholine, the coated silicon nanoparticles being associated with fluorouracil and, optionally, willow bark extract and/or an amino acid such as one or more of arginine and glycine) the waxy fatty acid esters are esters of decanoic acid (i.e. cetyl decanoate), and/or a mixture of stearyl heptanoate and stearyl caprylate. The composition may further comprise 1-hexadecanol. In certain preferred embodiments, the composition comprises a mixture of stearyl heptanoate, stearyl caprylate, and 1-hexadecanol. Preferably, the waxy fatty acid esters have emollient properties.

### Phase transition regulatory agents

### Examples: Limonene & Pluronic

The powder (for example, a powder comprising solid particles of one or more waxy fatty acid esters, into which are encapsulated silicon nanoparticles coated with one or more of phosphatidylcholine, hydrogenated phosphatidylcholine, the coated silicon nanoparticles being associated with fluorouracil and, optionally, willow bark extract and/or an amino acid such as one or more of arginine and glycine) may optionally comprise a terpene such as limonene and/or an alternative surfactant such as Pluronic (Poly(ethylene glycol)-block-poly(propylene glycol)-block-poly(ethylene glycol)). Limonene serves at least two roles. Firstly, it helps to regulate the phase transition temperature of the waxy fatty esters, thus exerting an effect in regulating the final melting point of the solid particles of the powder of the invention. It may also act as a penetration enhancer on skin and accelerate the rate of fluorouracil absorption. Other surfactants, such as pluronic (especially pluronic L-61) may also be used, preferably in addition to limonene rather than as a complete alternative. Limonene may also improve the shelf life and stability of products of the invention by virtue of its emulsifier properties. It is preferred to use (R)-(+)-Limonene (~90%). Other less purified forms of limonene, such as essential citrus oils, may also be used, but may be required at higher concentrations to achieve the same effect.

### Topical Creams and Gels

According to a third aspect, the invention provides a pharmaceutically compatible cream or gel suitable for topical application to the skin or other body surface, comprising a cream base into which a pharmaceutically compatible powder according to the second aspect of the invention is suspended (for example, a powder comprising solid particles of one or more waxy fatty acid esters, into which are encapsulated silicon nanoparticles coated with one or more of phosphatidylcholine, hydrogenated phosphatidylcholine, the coated silicon nanoparticles being associated with fluorouracil and, optionally, willow bark extract and/or an amino acid such as one or more of arginine and glycine). FDA and EMA guidelines with respect to maximum levels of fluorouracil in topical formulations specify 5% by total weight as the maximum recommended level. Therefore, according to preferred embodiments, topical creams and gels comprise up to 5%, up to 6%, up to 4% up to 3%, up to 2%, up to 1% or up to 0.5% by weight of fluorouracil. Common dosages are 1%, 2% and 5% for treating basal cell carcinoma. The usual dosage for treating keratosis is 0.5%. Within the 5% dose regimen the recommended duration of therapy is 3 to 6 weeks; however, therapy may be required for as long as 10 to 12 weeks before lesions are obliterated.

A pharmaceutically compatible cream comprises a cream base. Cream bases are typically emulsions of water in oil or oil in water. Preferably, they are oil in water emulsions where the oil phase contains a mixture of lipids, sterols and emollients and also the majority (for example at least 50, 70 or 80%) of the powder of the second aspect of the invention. The terpene as mentioned above may substantially be found in the aqueous phase. Preferably, there is very little fluorouracil (for example less than 5% or less than 2% of the total fluorouracil by weight present) in the aqueous phase of a cream or gel and very little (for example less than 5% or less than 2% of the total fluorouracil by weight present) in the oil phase of a cream.

A pharmaceutically compatible gel comprises powder of the second aspect of the invention dispersed in the liquid phase of the oil. The gel is preferably a hydrogel (colloidal gel) comprising cross-linked polymers such as polyethylene oxide, polyacrylamides or agarose, methylcellulose, hyaluronan, elastin-like polypeptide, carbomer (polyacrylic acid), gelatin or collagen.

It may be preferred to use a gel having a hydrophilic matrix (for example a carbomer gel containing triethanolamine) because such gels can favour the rapid absorbance of fluorouracil once the waxy fatty ester encapsulation is ruptured and the fluorouracil comes into contact with the gel matrix.

The pharmaceutically compatible cream or gel of the third aspect of the invention may comprise between 0.05 and 5 % by weight fluorouracil, such as between 0.05 and 4 %, between 0.05 and 3 %, between 0.05 and 2 %, or between 0.05 and 1 % by weight fluorouracil. The pharmaceutically compatible cream or gel may comprise between 1 and 5 %, between 2 and 5 %, between 3 and 5 %, or between 4 and 5 % by weight fluorouracil. Optionally, the pharmaceutically compatible cream or gel further comprises between 0.5 and 20% by weight salicylates, such as between 5 and 15 %, between 6 and 14%, between 7 and 13%, or between 8 and 12 % by weight salicylates.

### Adhesive Patches

According to a fourth aspect, the invention provides an adhesive patch comprising a backing layer and an adhesive film, wherein the adhesive film comprises a pharmaceutically compatible powder according to the second aspect of the invention (for example, a powder comprising solid particles of one or more waxy fatty acid esters, into which are encapsulated silicon nanoparticles coated with one or more of phosphatidylcholine, hydrogenated phosphatidylcholine, the coated silicon nanoparticles being associated with fluorouracil and, optionally, willow bark extract and/or an amino acid such as one or more of arginine and glycine) or a cream or gel according to the third aspect of the invention (the cream or gel comprising a cream base into which a pharmaceutically compatible powder according to the second aspect of the invention is suspended).

A patch according to the invention is typically a transdermal patch and consists of a backing layer, which may be textile, polymer or paper and protects the patch from the outer environment; optionally a membrane, for example a polymer membrane which prevents migration of the fluorouracil through the backing layer; and an adhesive. The fluorouracil is preferably present in a powder in accordance with the second aspect of the invention, or a gel or cream in accordance with the third aspect of the invention. The fluorouracil-containing product may be provided in the adhesive layer or in a reservoir of the patch or when the fluorouracil is contained in a gel, the gel may act as a reservoir within the patch product (a so-called "monolithic" device). Preferably, the fluorouracil-containing product is present in the adhesive layer.

A patch can be useful in ensuring the correct dosage of a subject by decreasing the likelihood of incautious or inappropriate use by the final user. Moreover, a patch will limit the area treated, avoiding inadvertent spreading to other areas.

### Medical Treatments

Products of the invention (for example, nanoparticles coated with one or more of phosphatidylcholine, hydrogenated phosphatidylcholine, which are associated with fluorouracil, and which may also be associated with willow bark extract and/or an amino acid such as one or more of arginine and glycine) are suitable for use in treating diseases including superficial basal cell carcinoma, actinic keratoses, solar keratoses and scarring. Suitable scars for treatment include keloid scars, hypertrophic scars and scarring following surgery. Products of the invention can also be used to treat acne, in particular severe acne.

Preferred dosages (as a percentage of product weight) for basal cell carcinoma are 1%, 2% and 5%. Lower dosages, for example 0.25% to 1% or 0.1% to 0.5% may be suitable for other conditions, for example scarring.

### Combination Treatments

In addition to fluorouracil products, the invention may include one or more further active pharmaceutical ingredients, and methods of the invention may include the use of further active pharmaceutical ingredients (APIs). The further APIs may conveniently be co-formulated with the fluorouracil (for example, the further APIs may be co-formulated with fluorouracil for delivery via nanoparticles coated with one or more of phosphatidylcholine, hydrogenated phosphatidylcholine;
in such embodiments, the nanoparticles may also be associated with willow bark extract and/or an amino acid such as one or more of arginine and glycine). Especially preferred further APIs for basal cell carcinoma treatments include Imiquimod, Vismodegib and curcumin. Especially preferred further APIs for treatment of keratoses include Imiquimod, Ingenol mebutate, Diclofenac, retinoids (for example Adapalene, Tazarotene, retinol, isotretinoin, Acitretin and Tretinoin. Especially preferred further APIs for treatment of keloid scars include salicylic acid, corticosteroids and interferon. Especially preferred further APIs for treatment of acne include azelaic acid, benzoyl peroxide, salicylic acid, antibiotics, retinoids, nicotinamide and antihistamines, or alternatively their respective natural extracts of origin, i.e willow bark extract.

### Treatment Regimes

The products and methods of the invention (for example, products comprising nanoparticles coated with one or more of phosphatidylcholine, hydrogenated phosphatidylcholine, which are associated with fluorouracil, and which may also be associated with willow bark extract and/or an amino acid such as one or more of arginine and glycine) may be used in accordance with any dosage regime determined to be suitable. For example treatment may be continued until a disease is cured or until no further improvement accrues. A typical dosage course for treating keratosis lasts from 3 to 20 weeks, for example 3 to 12, 5 to 15 or 5 to 12 weeks. Similar regimes may be used for other conditions.

### Silicon-Containing Materials

As used herein, the term "a hydrolysable silicon-containing material" is any silicon-containing material which, upon administration to a human or animal subject, may be hydrolysed to OSA in a timely manner. Typically, 1 mg of nanoparticles of the hydrolysable silicon-containing material hydrolyses in 100 mL of physiological buffer, for example PBS, within one hour at 37 °C. The silicon-containing materials of the present invention comprise at least 50 wt% silicon. For example, the silicon-containing materials of the present invention may comprise at least 70 wt% silicon. The silicon-containing materials may be substantially pure silicon, for example, materials comprising at least 90 wt% silicon, preferably at least 95 wt% silicon, especially at least 99 wt% silicon. The hydrolysable silicon-containing material is typically a semiconductor material such as amorphous silicon. Semiconductor grade silicon typically comprises very high purity silicon, for example at least 99.99 wt%. Substantially pure silicon materials may, optionally, include trace amounts of other elements, such as boron, arsenic, phosphorus and/or gallium, for example, as semiconductor doping agents. The substantially pure silicon material may be a P-type doped silicon wafer, for example, containing trace amounts of boron or another group III element, or N-type silicon wafers, for example containing trace amounts of phosphorous or another group VI element. The surface of the silicon material typically includes silanol (Si-OH) groups. Suitable hydrolysable silicon-containing materials for use according to the invention include but are not limited to nanosilicon (single or polycrystal), of semi conductive grade and nanosilicon.

Suitably, the silicon content of the products of the invention (for example, products comprising nanoparticles coated with one or more of phosphatidylcholine, hydrogenated phosphatidylcholine, which are associated with fluorouracil, and which may also be associated with willow bark extract and/or an amino acid such as one or more of arginine and glycine) is within the range of 0.01-50 wt %, preferably within the range of 0.01-10 wt%, more preferably within the range of 0.1-10 wt%, and most preferably within the range of 0.1-5 wt%. In one embodiment, the silicon content of the composition is in the range of from 1 wt% to 30 wt%, for example from 2 wt% to 20 wt%, preferably from 3 wt% to 15 wt% based on the total weight of the composition.

### Nanoparticles

For the purposes of this invention, the term "nanoparticle" is typically used to describe a particle having at least one dimension in the nanometre range, i.e. of 300 nm or less and having the same behaviours and properties as nanoparticles. The nanoparticles for use according to the invention (for example, nanoparticles coated with one or more of phosphatidylcholine, hydrogenated phosphatidylcholine, , which are associated with fluorouracil, and which may also be associated with willow bark extract and/or an amino acid such as one or more of arginine and glycine) typically have an average particle diameter of less than 300 nm, preferably less than 200 nm and especially less than 100 nm. In one embodiment, the nanoparticles have an average particle diameter in the range of from 10 to 100 nm, preferably from 20 to 80 nm and especially from 10 to 50 nm. In other embodiments, the nanoparticles have an average particle diameter of from 50 to 200 nm, 60 to 250 nm or 80 to 240 nm. In preferred embodiments (for example, when the silicon nanoparticles are coated with one or more of phosphatidylcholine, hydrogenated phosphatidylcholine, these nanoparticles being associated with fluorouracil and optionally with willow bark extract and/or an amino acid such as one or more of arginine and glycine) the nanoparticles have an average particle diameter of from 30 to 100 nm.The average particle diameter is the average maximum particle dimension, it being understood that the particles are not necessarily spherical. The particle size may conveniently be measured using conventional techniques such as microscopy techniques for example scanning electron microscopy.

In some embodiments, the silicon particles for use according to the invention (for example, silicon particles coated with one or more of phosphatidylcholine, hydrogenated phosphatidylcholine, which are associated with fluorouracil, and which may also be associated with willow bark extract and/or an amino acid such as one or more of arginine and glycine) may have an average particle diameter of less than 1000 µm, for example from 1 to 1000 µm, from 100 to 1000 µm, or from 500 to 1000 µm. The silicon particles may have an average particle diameter of less than 500 µm, for example from 1 to 500 µm or from 100 to 500 µm. The silicon particles may have an average particle diameter of less than 50 µm, for example from 1 to 50 µm or from 25 to 50 µm. The silicon particles may have an average particle diameter of less than 10 µm, for example from 1 to 10 µm, or from 5 to 10 µm.

In some embodiments (for example, when the silicon nanoparticles are coated with one or more of phosphatidylcholine, hydrogenated phosphatidylcholine, these nanoparticles being associated with fluorouracil and optionally with willow bark extract and/or an amino acid such as one or more of arginine and glycine) the nanoparticles relating to the invention have a spherical or substantially spherical shape. The shape may conveniently be assessed by conventional light or electron microscopy techniques.

### Preparation of silicon-containing nanoparticles

The silicon-containing nanoparticles relating to the invention may conveniently be prepared by techniques conventional in the art, for example by milling processes or by other known techniques for particle size reduction. The silicon-containing nanoparticles made from sodium silicate particle, colloidal silica or silicon wafer materials. Macro or micro scale particles are ground in a ball mill, a planetary ball mill, plasma or laser ablation methods or other size reducing mechanism. The resulting particles are air classified to recover nanoparticles. It is also possible to use plasma methods and laser ablation for nanoparticles production.

Porous nanoparticles may be prepared by methods conventional in the art, including the methods described herein.

### Addition of Phospholipid

Prior to the addition of a stabilizing phospholipid (for example, one or more of phosphatidylcholine, hydrogenated phosphatidylcholine, phosphatidylethanolamine, components of lecithin, particularly one or more of phosphatidylcholine, hydrogenated phosphatidylcholine) the porous nanoparticle is preferably "activated" in order to improve adhesion of the phospholipid. Activation may be carried out by any suitable means. For example, the porous nanoparticle may be washed with a volatile solvent (for example, ethanol, methanol, acetone or xylene) which is then allowed to evaporate. Alternatively, the porous nanoparticle may be washed with a volatile solvent which is miscible with water (for example an alcohol such as ethanol), and then washed in water and dried of the water by a freeze drying step.

The phospholipid may then be added to the activated nanoparticles. Preferably, this is done by dissolving the phospholipid in a volatile solvent such as an alcohol like methanol and ethanol, mixing this with the nanoparticles and then allowing the solvent to evaporate (for example using a rotary evaporation system) whilst the particles are agitated.

### Preparation of Powder

The powder is made by placing the phospholipid coated nanoparticles (for example, nanoparticles coated with one or more of phosphatidylcholine, hydrogenated phosphatidylcholine, phosphatidylethanolamine, components of lecithin, phosphatidylcholine) in a molten waxy fatty acid ester or mixture thereof (preferably at no more than 30 °C, 35 °C, 37 °C , 40 °C 45 °C, 50 °C or 55 °C) and mixing. The waxy fatty acid ester is then transformed into a powder by any suitable means, for example by solidifying and then milling or by emulsification and then solidification. The addition of a terpene such as limonene may assist in emulsification.

The terpene is also able to assist the phase transition state of the overall formulation. Several lipids that may constitute the molten waxy fatty acid ester or mixture thereof are not able to melt once applied on skin (i.e. 1-hexadecanol). The use of terpenes favors the melting of these particles once applied on skin by means of body temperature/friction caused by rubbing the powder on skin.

### Preparation of Creams and Gels

Creams and gels may be formulated simply by dispersing (i.e. mixing) the powder with a cream or gel base. For example, the powder may be stirred into a pharmaceutical cream base. In respect of a gel, the powder may be stirred into the gel matrix in powder form and then the gel hydrated, or it may be stirred into a pre-hydrated gel.

### Preparation of Patches

A patch may be formulated by any appropriate method, for example, a patch containing a muco-adhesive hydrophilic gel may be produced, the gel may be produced with the powder of the invention, dispersed in it and the gel may optionally be dried by gentle evaporation of water to become a film with the required adhesive properties.

### EXAMPLES

The invention may be further illustrated by the following non-limiting examples.

### Materials

Distilled water, Cetyl decanoate, limonene, sodium bicarbonate, 5-Fluorouracil (5FU), 1-hexadecanol, activated silicon nanoparticles (SiNPs, 100nm), hydrogenated phosphatidylcholine (PHOSPHOLIPON 90 G, yellowish wax - hydrogenated phosphatidylcholine fully soluble in EtOH only), distilled water, ethanol.

### Silicon Preparation

Single-side polished P-type or N-type silicon wafers were purchased from Si-Mat, Germany. All cleaning and etching reagents were clean room grade. A heavily doped P þþ type Si(100) wafer with a resistivity of 0.005 V cm⁻¹ was used as the substrate. A 200-nm layer of silicon nitride was deposited by a low-pressure chemical vapour deposition system. Standard photolithography was used to pattern using an EVG 620 contact aligner. Porous nanoparticles were formed in a mixture of hydrofluoric acid (HF) and ethanol (3:7 v/v) by applying a current density of 80 mA cm⁻² for 25 s. A high-porosity layer was formed by applying a current density of 320 mA cm⁻² for 6 s in a 49% HF:ethanol mixture with a ratio of 2:5 (v/v). Smaller pores can be formed in a mixture of HF (49%) and ethanol (3:7 v/v) by applying a current density of 80 mA cm 22 for 25 s. In the specific case, pores were formed in a mixture of HF (49%) and ethanol (1:1 v/v) by applying a current density of 6 mA cm⁻² for 1.75 min. After removing the nitride layer by HF, particles were released by ultrasound in isopropyl alcohol for 1 min. The shape, which is mainly hemispherical, is determined by means of scanning electron micrograph (SEM). The size of pores can be determined by means of nitrogen adsorption-desorption volumetric isotherms. After etching, the samples were rinsed with pure ethanol and dried under a stream of dry high-purity nitrogen prior to use.

Etched Silicon wafers, P+ or N- were crushed using a ball mill and/or pestle & mortar. The fine powder sieved using Retsch branded sieve gauge 38 µm and shaker AS200. Uniformity at the selected sizes (20-100 µm) is achieved by the aperture size of the sieve. The particle sizes were measured by the Quantachrome system and PCS from Malvern Instruments. Samples were kept in the closed container until further use.

NanoSilicon powder was also obtained from Sigma and Hefel Kaier, China. The particle size measured by PCS and recorded (size was range between 20-100nm) before subjected to the loading and etching. Silicon wafers were crushed using a ball mill, or using mortar and pestle. The fine powder was sieved using a Retsch branded sieve gauge 38 µm and shaker AS200 and uniform nanoparticles of the desired size were collected.

### Activation of Silicon Nanoparticles

250 mL of ethanol and 500 mg of 30-100 nm diameter porous silicon nanoparticles were mixed and stirred for 30 minutes. The solution was then centrifuged for 30 minutes at 3000 rpm. The supernatant was discarded and the nanoparticles washed in 5 mL of distilled water and transferred to a round bottomed flask. The contents of the flask were frozen (2 hours at -25 °C). The frozen nanoparticles were freeze-dried using a freeze dryer overnight. The resultant dry powder is the activated silicon nanoparticles.

Alternatively, 250 mL of methanol and 500 g of 30 nm diameter porous silicon nanoparticles were mixed and stirred for 120 minutes. The obtained paste was transferred onto specific trays for dehydration, in order to completely evaporate the organic solvent residue (24hrs, room temperature). Once a solid thin layer was obtained, this layer was crushed and milled until a powder was obtained. The resultant dry powder is the activated silicon nanoparticles.

### Stabilization with Bilayer Film of Hydrogenated Phosphatidylcholine

150 mg of hydrogenated phosphatidylcholine in 30 ml ethanol was prepared. The flask was connected to a rotary evaporation system at 45 °C until the sample was dry (at least 5 minutes).

### Rehydration of Liposome and Loading with Fluorouracil

15 mg of stabilized nanoparticle was transferred to a beaker, to which 300 mg of fluorouracil was also added. 20 mL of distilled water was added to the mixture and the contents of the beaker homogenized by sonication for 5 minutes at 30 °C and then vortexing.

### Drying of Fluorouracil Loading Stabilized Particles

The solution obtained in the previous method was cooled in a fridge (4 °C for at least 2 hours) and then frozen (-20 °C for at least 4 hours). The frozen solution was freeze dried overnight to obtain a powder and was stored in a fridge until further use. These stabilized particles can be directly dispersed into an appropriate gel or optionally further coated for modifying the kinetics of API release. Optionally, the particles can be further associated with willow bark extract (see below for a protocol wherein particles according to the invention are further associated with willow bark extract).

### Production of Powder Containing Fluorouracil Nanoparticles

1.00 g of 1-hexadecanol and 0.7 g of cetyl decanoate was transferred to a tall form 250 mL beaker. Fluorouracil-loaded particle powder (i.e. prepared as above) was added to the beaker. In a separate beaker, 120 mL of distilled water was boiled, to which 1.0 g of sodium bicarbonate was added, together with 2mL of phase transition regulator agent. The beaker containing the cetyl decanoate in 1-hexadecanol as well as 5FU was heated until the contents melted to an oily liquid. A Polimix rotary mixer was prepared with ice cubes and acetone as a cooling mixture in its surrounding jacket. The oily liquid mixture was transferred to a beaker and placed into the Polimix mixer at 930 rpm. The boiled sodium bicarbonate/phase transition regulatory agent solution was added to the oily liquid mixture. After 30 seconds the mixer speed was set to 830 rpm and left for 15 minutes with external cooling. The resultant powder was then filtered out of the solution and allowed to dry for 5 to 6 days.

### Manufacture of Patches

- Disperse 1.0g of Hypromellose powder in 40mL of distilled water previously warmed up, then put the beaker on the hotplate (T=40 °C, magnetic stirring rpm=7) for 3h.
- When the resulting suspension appears opalescent, take the gel out of the hotplate, without any magnetic stirring, and leave the sample to cold down at room temperature, then move to the fridge and leave it there overnight.
- Once reached a temperature of 4 °C, add 0.25g of Pluronic.
- Mix the obtained mixture gently and add purified water up to 50mL.
- Keep the sample in the fridge until use. Please note that this gel needs to be diluted with another 50ml of formula containing an appropriate amount of 5FU. Weigh the required amount of a powder of the invention and gently disperse into 15 mL of the gel. Homogenize the obtained mixture to ensure uniformly dispersed microspheres in the gel.
- The final concentration of the gel is [1.0% Hypromellose and 0.25% Pluronic L-61].

### Method

- Weigh 0.05 g EDTA and disperse into 20 mL water (previously warmed up to 60°C) into an appropriate beaker. Stir until complete solubilization.
- Weigh 0.05 g of PVP (polyvinylpyrrolidone) K90 and disperse in the above solution. Stir until completely dissolved.
- Weigh 0.80 g Natrosol (hydroxyethyl cellulose) and disperse in the above solution. gently stir.
- Weigh 0.15 g trehalose and disperse in the above solution. Stir gently until a homogeneous mass is formed.
- When the product has reached room temperature, add 15 mL distilled water and 0.5 mL limonene, and then gently stir.
- Sonicate above solution for 2 hrs.
- Weigh 5.0 g of the above viscous solution into a beaker
- Add 0.4 g of powder according to the invention prepared as described above loaded with fluorouracil, to the obtained viscous gel, then stir gently.
- Transfer the obtained viscous solution mixed with the powder into a silicone slot mould (4.5cm x 4.5cm), then move into a thermostatic chamber (30 °C, 15-35% RE) for 20 hrs.

The obtained film is a mucoadhesive film loaded with the powder of the invention, ready to be applied onto skin and which may be further provided with a suitable backing layer.

### Preparation of silicon nanoparticles associated with willow bark extract

An exemplary protocol for preparing nanoparticles comprising 0.5 wt % fluorouracil and 10 wt % willow bark extract loaded nanoparticles is as follows.

### Materials

| **Fluorouracil (mg)** | **Willow Bark (ml)** | **Silicon nanoparticles (activated) (mg)** | **phosphatidylcholine (mg)** | **Arginine (mg)** | **Glycine (mg)** |
|---|---|---|---|---|---|
| 1500 | 30 | 16 | 624 | 4 | 2 |

### Preparation of hydrogenated phosphatidylcholine (PC) stock solution (solution A)

- Dissolve 624 mg of PC in 250 mL of ethanol and sonicate. The final concentration is 2.5 mg/mL.

### Preparation of solution for rehydration of PC-Willow Bark dry foam (solution B)

- Add 16 mg of activated silicon nanoparticles (SiNPs) (30 nm) to a beaker.
- Add 4 mg of arginine to the beaker. Then add 2 mg of glycine to the beaker.
- Add 1500 mg of fluorouracil to the same beaker containing SiNPs, arginine and glycine.
- Disperse this mixture in 200 mL distilled water by stirring for 15 minutes.

### Lipid-based thin film PC-Willow Bark dry foam formation using phosphatidylcholine and Willow Bark extract

- Dissolve 624 mg of hydrogenated phosphatidylcholine in 250 mL of ethanol, then sonicate for at least 10 minutes at 45 °C in a water bath. Then, move this mixture to a round bottomed flask.
- Add 30 mL of willow bark extract to the round bottomed flask.
- Connect the round bottomed flask to a rotary evaporation system.
- Maintain rotary evaporation at the maximum speed rate for 45 minutes (at room temperature).
- Lower the temperature to - 45 °C. Leave the sample to dry for at least for 15 minutes.
- The product will appear as a thick white foam.

### Rehydration of PC-Willow Bark dry lipid-based foam

- Add 16 mg of activated silicon nanoparticles (SiNPs, size 30 nm) to a beaker.
- Add 4 mg of arginine to the beaker. Then add 2 mg of Glycine to the same beaker.
- Add 1500 mg of fluorouracil to the same beaker containing the SiNPs, arginine and glycine.
- Disperse the mixture in 120 ml distilled water by sonicating for 5 minutes at 30 °C.
- Vortex the solution to homogenise the components.
- Add the solution to the round bottomed flask which contains the dry foam (PC and willow bark extract) formulation. Vortex until the foam is fully dissolved.
- Wash the round bottom flask with 10 ml of distilled water.
- Sonicate the obtained dissolved foam (130 ml total amount) for 30 minutes at 30 °C.
- Place in the fridge for 1 hour, then move to the freezer for circa 3 hours at (-25°C).
- Connect the tubes to a freeze-dryer device for at least 3 days to obtain a dry powder by evaporating the solvent.

The obtained powder can be stored for reconstitution with purified water and mixing with an appropriate vehicle. Optionally, the freeze drying step can be omitted, and the sonicated dissolved foam can be mixed directly with the intended vehicle.

### Preparation of gel for dispersion of final product

### Gel materials

| **Aqueous Phase** | **EDTA** | **Hypromellose** | **Pluronic L-61** | **Distilled Water** |
|---|---|---|---|---|
| **Gel** | 0.05g | 1.00g | 0.25g | up to 50g |

- Disperse 1.0 g of Hypromellose powder in a beaker of 40 mL of distilled water. Place the beaker on the hotplate (40 °C, magnetic stirring rpm 7) for 3 hours.
- When the resulting suspension appears opalescent, remove the gel from the hotplate, cease stirring, and leave the sample to cool to room temperature. Transfer to a fridge (4 °C) overnight.
- Once the gel has cooled to 4 °C, add 0.25 g of Pluronic L-61. Pluronic L-61 is a masking agent with a cloudy point in the range 20-24 °C.
- Mix the obtained mixture gently and add distilled water up to 50 mL.
- Keep the sample in the fridge until use. This gel needs to be diluted with either 50 ml of pure solvent or 50 mL of a silicon nanoparticle suspension, to reach a concentration equal to 1.0% Hypromellose and 0.25% Pluronic L-61.

### Preparation of the final product

- Disperse the powder in 150 mL of distilled water. The powder contains willow bark extract having the equivalent of 3 g salicylic acid; 16 mg silicon nanoparticles; 624 mg PC; 1500 mg 5-fluorouracil; 4 mg arginine; and 2 mg glycine, in 150 mL of distilled water.
- Add 150 g of the gel to this dispersion.
- Vortex the mixture for 20 minutes to homogenise.
- Store the final product at 4°C.

### Further examples

For the examples below, silicon nanoparticles were prepared associated with lipid (PC), willow bark extract, arginine, glycine, fluorouracil, and a gel comprising Hypromellose and Pluronic L-61, as indicated in the protocol above. This formulation was dispersed with EDTA in distilled water.

### Cytotoxicity assay of silicon nanoparticles associated with fluorouracil and willow bark

An assay was prepared to test the cytotoxicity of the formulation. The results are shown below, showing 100% cell lysis. This confirmed the retention of normal biological activity of fluorouracil when associated with the nanoparticles of the invention.

| **Time** | **Test Article** | **Controls** | | |
|---|---|---|---|---|
| | **100%** | **Vehicle** | **Negative** | **Positive** |
| **24 Hours** | 4 | 0 | 0 | 4 |
| | 4 | 0 | 0 | 4 |
| | 4 | 0 | 0 | 4 |
| **Grade Average** | 4 | 0 | 0 | 4 |

### Preservative effectiveness testing (PET)

Formulations were tested in preservative effectiveness testing meeting the current United States Pharmacopeia (USP) <51> category II antimicrobial preservatives effectiveness test and USP <61> suitability testing. The test includes the following pathogen growth tests for bacteria, yeast and mould. Group 1 *S*. *aureus* ATCC 6583; Group II *P. aeruginosa* ATCC 9027; Group III *A*. *brasiliensis* ATCC 16404; Group IV *C*. *albicans* ATCC 10231; Group V *E. coli ATCC* 8739. The results indicated a PET pass result for bacteria and yeast / mould counts.

| | **Log reduction** | | | |
|---|---|---|---|---|
| **Group** | **Day 7** | **Day 14** | **Day 28** | **Pass / Fail** |
| **Group 1, *S*. *aureus* ATCC 6583** | 4.74 | 4.74 | 4.74 | Pass |
| **Group II, *P. aeruginosa* ATCC 9027** | 4.39 | 4.39 | 4.39 | Pass |
| **Group III, *A. brasiliensis* ATCC 16404** | 0.69 | 0.83 | 1.05 | Pass |
| **Group IV, *C. albicans* ATCC 10231** | 4.56 | 4.56 | 4.56 | Pass |
| **Group V, *E. coli* ATCC 8739** | 4.83 | 4.83 | 4.83 | Pass |

### Dermal sensitization test in guinea pigs (GLP study)

A Magnusson-Kligman sensitization test on guinea pigs was conducted to determine whether the fluorouracil-associated nanoparticles of the present invention provoke a dermal skin sensitization reaction. The study included an intradermal and topical induction phase and a challenge phase. The testing met the following standards: American National Standards Institute/ Association for the Advancement of Medical Instrumentation/ International Organization for Standardization (ANSI/AAMI/ISO) 10993-1 - Biological evaluation of medical devices - Part 2: Animal welfare requirements; and ANSI/AAMI/ISO 10993-10 - Biological evaluation of medical devices - Part 10: Tests for irritation and skin sensitization.

The results demonstrated no irritation at any test site, either at 24 or at 48 hours after the challenge patch removal. Based on these findings and on the evaluation-system used the nanoparticles of the invention, formulated with fluorouracil, are not considered to be a contact sensitizer.

### Scoring system for dermal sensitization test

| **Magnusson and Kligman Scale (ANSI/AAMI/ISO 10993-10)** | |
|---|---|
| **Patch Test Reaction** | Grading Scale |
| **No visible change** | 0 |
| **Discrete or patchy erythema** | 1 |
| **Moderate and confluent erythema** | 2 |
| **Intense erythema and / or swelling** | 3 |

### Dermal sensitization test results

| Animal No. (with nanoparticles) | Hours after patch removal | | Animal No. (with positive control, Hexyl Cinnamic Aldehyde) | Hours after patch removal | | Animal No. (with negative control) | Hours after patch removal | |
|---|---|---|---|---|---|---|---|---|
| | 24 | 48 | | 24 | 48 | | 24 | 48 |
| 3601 | 0 | 0 | 3701 | 0.5 | 0.5 | 3711 | 0 | 0 |
| 3602 | 0 | 0 | 3702 | 2 | 2 | 3712 | 0 | 0 |
| 3603 | 0 | 0 | 3703 | 1 | 0.5 | 3713 | 0.5 | 0 |
| 3604 | 0 | 0 | 3704 | 0.5 | 0.5 | 3714 | 0 | 0 |
| 3605 | 0 | 0 | 3705 | 1 | 1 | 3715 | 0 | 0 |
| 3606 | 0 | 0 | 3706 | 1 | 1 | - | | |
| 3607 | 0 | 0 | 3707 | 1 | 2 | - | | |
| 3608 | 0 | 0 | 3708 | 1 | 1 | - | | |
| 3609 | 0 | 0 | 3709 | 1 | 1 | - | | |
| 3610 | 0 | 0 | 3710 | 1 | 2 | - | | |

### Dermal irritation / sensitization evaluation clinical safety studies in humans (Repeat Insult Patch Test - RIPT)

All the human dermal trials are double-blind studies performed in human patients (n = 52). In the human subject testing for safety (Repeat Insult Patch Test - RIPT) in relation to skin irritation / sensitization evaluation, 0.2ml of test material was dispensed directly onto the designated area of the subject's skin and allowed to air-dry. This was repeated until a series of 9 consecutive patch areas were applied, on three days per week for three weeks. Subjects were then given a 10 to 14 day rest period before further application of the material with assessments at a further 24 and 48 hour period.

The scoring system was as follows:
0 - No evidence of any effect
0.5 - (Barely perceptible) minimal faint (light pink) uniform or spotty erythema
1 - (Mild) pink uniform erythema covering most of contact site
2 - (Moderate) pink/red erythema visibly uniform in entire contact area
3 - (Marked) bright red erythema with accompanying edema petechiae or papules
4 - (Severe) deep red erythema with vesiculation or weeping with or without edema

In the 24 hour patch test skin irritation evaluation with an occlusive patch, all 52 subjects had a score of zero (0) and there were no adverse reactions of any kind during the course of the study, and no instances of erythema. The test material formulated according to the invention is therefore considered to be a 'non-primary irritant' when applied to the skin.

For the repeat insulting patch test (RIPT) skin irritation/sensitization evaluation all 52 subjects had a score of zero (0) at the evaluated time-points of 0h, 24h and 48h. There were no adverse reactions of any kind during the course of the study. The test material formulated according to the invention is therefore considered to be a 'non-primary irritant' and 'non-primary sensitizer' to the skin in humans.

### In vitro permeation test of the activity of silicon nanoparticles associated with fluorouracil and willow bark

Nanoparticles according to the invention were prepared, associated with fluorouracil and/or willow bark extract in varying amounts. Three such formulations were prepared, see Table 1. As a control sample, Efudex cream was used, comprising 5 wt % fluorouracil, Stearyl alcohol, white soft paraffin, polysorbate 60, propylene glycol, methyl parahydroxybenzoate, propyl parahydroxybenzoate and purified water.

**Table 1 - Test samples**

| Sample | Fluorouracil (wt %) | Willow bark extract (wt%) |
|---|---|---|
| Efudex | 5.00 | 0.00 |
| S1 | 5.00 | 0.00 |
| S2 | 0.50 | 10.00 |
| S3 | 0.50 | 0.00 |

An *in vitro* permeation test (IVPT) was used to examine the permeation profile of each sample through human skin over the course of 24 hours. Table 2 below shows the amount of fluorouracil detected in the receptor fluid over time, i.e. the amount of fluorouracil which passed through the skin membrane between the donor chamber and the receptor chamber during IVPT. In Table 2, b.l.q. stands for below the limit of quantification.

**Table 2 - permeation of fluorouracil into the receptor fluid over time during IVPT**

| Time (h) | Efudex | S1 | S2 | S3 |
|---|---|---|---|---|
| 0.25 | b.l.q. | b.l.q. | b.l.q. | b.l.q. |
| 1 | b.l.q. | b.l.q. | b.l.q. | b.l.q. |
| 2 | b.l.q. | b.l.q. | b.l.q. | b.l.q. |
| 4 | b.l.q. | b.l.q. | b.l.q. | b.l.q. |
| 22 | 41.637 µg | b.l.q. | 9.927 µg | b.l.q. |
| 24 | 54.538 µg | b.l.q. | 1.145 µg | b.l.q. |
| Total % permeated | 7.090 | - | 1.466 | - |

As shown in Table 2, fluorouracil suspended in a conventional Efudex cream is able to pass through the skin membrane with ease. However, fluorouracil associated with the nanoparticles of the present invention is delivered to the skin in a much more controlled manner, and does not pass through the skin in this way. Where willow bark is used in association with the nanoparticles of the invention, delivery to the skin is still controlled (compared to Efudex) but penetration into each of the layers of the skin occurs at a slightly higher rate compared to nanoparticles of the invention without willow bark.

The permeation profile of each sample was analysed at the end of 24 hours, at each layer of the skin. The results are shown in Table 3.

**Table 3 - Permeation of fluorouracil into the skin layers after 24 hours' IVPT**

| Skin layer | Efudex | S1 | S2 | S3 |
|---|---|---|---|---|
| Stratum corneum | b.l.q. | b.l.q. | b.l.q. | b.l.q. |
| Epidermis | b.l.q. | 1.555% | 5.622% | b.l.q. |
| Dermis | b.l.q. | b.l.q. | 9.505% | b.l.q. |
| Receptor fluid | 7.090 % | 1.555% | 1.466% | b.l.q. |

As shown by Table 3, fluorouracil suspended in a conventional Efudex cream (5 wt % fluorouracil) passes through the skin membrane without being trapped in any skin layer. When fluorouracil is associated with the nanoparticles of the present invention (S1, 5 wt % fluorouracil) its release is more controlled, and a smaller amount is released into the epidermis, with no fluorouracil reaching the receptor fluid. At lower concentrations of fluorouracil (S3, 0.5 wt % fluorouracil) no fluorouracil release is observed. However, when willow bark extract is associated with the nanoparticles of the invention (S2, 0.5 wt % fluorouracil) release of fluorouracil is seen into each of the layers of the skin in a controlled manner, with little fluorouracil passing to the receptor fluid.

### In vitro Franz cell permeation assay

The conventional Efudex cream (5 wt % fluorouracil) was also compared to S2 (10 wt % willow bark extract, 0.5 wt % fluorouracil) in an in vitro Franz cell permeation assay. After 24 hours, tissue samples were harvested and skin tissue layers were separated followed by fluorouracil extraction and bioanalytical quantification of the drug to determine the extent of localization of fluorouracil within skin tissue layers and permeation of the drug through the skin tissue samples. Tissue samples examined included samples collected from the stratum corneum, the epidermis, and the dermis.

In the case of Efudex, no fluorouracil was found to be present in the stratum corneum, epidermis or dermis. 3.55 % of the fluorouracil applied in the Efudex cream was found to have permeated entirely through the skin layers. This suggests that with conventional Efudex cream, any fluorouracil that does pass through the stratum corneum quickly passes through the remaining skin layers in an uncontrolled manner.

In the case of S2, no fluorouracil was found in the stratum corneum or the epidermis. However, 10.13 % of the fluorouracil applied in S2 was found to be present in the dermis, while only 0.73 % had permeated entirely through the skin. This suggests that when fluorouracil is applied which is associated with the nanoparticles of the invention, it passes through the skin in a more controlled manner compared to conventional creams (such as Efudex) which merely comprise suspended molecules of fluorouracil dispersed in a cream base.

## Claims

1. Pharmaceutically compatible nanoparticles comprising at least 50% by weight hydrolysable silicon, wherein the nanoparticles are surface coated with a phospholipid, and wherein the coated nanoparticles are associated with fluorouracil.

2. The pharmaceutically compatible nanoparticles of claim 1, wherein the nanoparticles are associated with willow bark extract.

3. The pharmaceutically compatible nanoparticles of claim 1 or claim 2, wherein the phospholipid comprises one or more of phosphatidylcholine, hydrogenated phosphatidylcholine, phosphatidylethanolamine, a component of lecithin, a phosphoinositide, or a phosphosphingolipid.

4. The pharmaceutically compatible nanoparticles of any one of the preceding claims, wherein the pharmaceutically compatible nanoparticles are porous.

5. The pharmaceutically compatible nanoparticles of any one of the preceding claims, wherein the phospholipid coating comprises a bilayer of phosphatidylcholine.

6. The pharmaceutically compatible nanoparticles of any one of the preceding claims, wherein the nanoparticles are associated with one or more amino acids.

7. The pharmaceutically compatible nanoparticles of claim 6, wherein the one or more amino acids are selected from arginine and glycine.

8. The pharmaceutically compatible nanoparticles of any one of claims 1 to 7, wherein at least 90% of the fluorouracil is physically associated with or absorbed onto the surface of the silicon nanoparticles and/or a phospholipid bilayer.

9. The pharmaceutically compatible nanoparticles of any one of claims 1 to 8, for use as a medicament.

10. The pharmaceutically compatible nanoparticles of any one of claims 1 to 8, for use in the treatment of superficial basal cell carcinoma, actinic keratoses, solar keratoses, acne or scarring.

11. The pharmaceutically compatible nanoparticles for use of claim 10, wherein the scarring is selected from one or more of keloid scarring, hypertrophic scarring, or scarring following surgery.

12. The pharmaceutically compatible nanoparticles for use of claim 11, wherein the scarring is hypertrophic scarring.

## Patentansprüche

1. Pharmazeutisch verträgliche Nanopartikel, umfassend mindestens 50 Gew.-% hydrolysierbares Silicium, wobei die Nanopartikel mit einem Phospholipid oberflächenbeschichtet sind und wobei die beschichteten Nanopartikel mit Fluoruracil assoziiert sind.

2. Pharmazeutisch verträgliche Nanopartikel nach Anspruch 1, wobei die Nanopartikel mit Weidenrindenextrakt assoziiert sind.

3. Pharmazeutisch verträgliche Nanopartikel nach Anspruch 1 oder Anspruch 2, wobei das Phospholipid eines oder mehrere von Phosphatidylcholin, hydriertem Phosphatidylcholin, Phosphatidylethanolamin, einem Bestandteil von Lecithin, einem Phosphoinositid oder einem Phosphosphingolipid umfasst.

4. Pharmazeutisch verträgliche Nanopartikel nach einem der vorhergehenden Ansprüche, wobei die pharmazeutisch verträglichen Nanopartikel porös sind.

5. Pharmazeutisch verträgliche Nanopartikel nach einem der vorhergehenden Ansprüche, wobei die Phospholipidbeschichtung eine Doppelschicht aus Phosphatidylcholin umfasst.

6. Pharmazeutisch verträgliche Nanopartikel nach einem der vorhergehenden Ansprüche, wobei die Nanopartikel mit einer oder mehreren Aminosäuren assoziiert sind.

7. Pharmazeutisch verträgliche Nanopartikel nach Anspruch 6, wobei die eine oder die mehreren Aminosäuren aus Arginin und Glycin ausgewählt sind.

8. Pharmazeutisch verträgliche Nanopartikel nach einem der Ansprüche 1 bis 7, wobei mindestens 90 % des Fluoruracils physikalisch an der Oberfläche der Silicium-Nanopartikel und/oder einer Phospholipid-Doppelschicht assoziiert oder daran absorbiert sind.

9. Pharmazeutisch verträgliche Nanopartikel nach einem der Ansprüche 1 bis 8 zur Verwendung als Arzneimittel.

10. Pharmazeutisch verträgliche Nanopartikel nach einem der Ansprüche 1 bis 8 zur Verwendung bei der Behandlung von oberflächlichem Basalzellkarzinom, aktinischen Keratosen, solaren Keratosen, Akne oder Narbenbildung.

11. Pharmazeutisch verträgliche Nanopartikel zur Verwendung nach Anspruch 10, wobei die Narbenbildung aus einer oder mehreren von Keloid Narbenbildung, hypertropher Narbenbildung oder Narbenbildung nach einem chirurgischen Eingriff ausgewählt ist.

12. Pharmazeutisch verträgliche Nanopartikel zur Verwendung nach Anspruch 11, wobei die Narbenbildung eine hypertrophe Narbenbildung ist.

## Revendications

1. Nanoparticules pharmaceutiquement compatibles comprenant au moins 50 % en poids de silicium hydrolysable, dans lesquelles les nanoparticules sont revêtues en surface d'un phospholipide, et dans lesquelles les nanoparticules revêtues sont associées au fluorouracile.

2. Nanoparticules pharmaceutiquement compatibles selon la revendication 1, dans lesquelles les nanoparticules sont associées à un extrait d'écorce de saule.

3. Nanoparticules pharmaceutiquement compatibles selon la revendication 1 ou la revendication 2, dans lesquelles le phospholipide comprend un ou plusieurs parmi la phosphatidylcholine, la phosphatidylcholine hydrogénée, la phosphatidyléthanolamine, un composant de lécithine, un phosphoinositide ou un phosphosphingolipide.

4. Nanoparticules pharmaceutiquement compatibles selon l'une quelconque des revendications précédentes, dans lesquelles les nanoparticules pharmaceutiquement compatibles sont poreuses.

5. Nanoparticules pharmaceutiquement compatibles selon l'une quelconque des revendications précédentes, dans lesquelles le revêtement de phospholipide comprend une bicouche de phosphatidylcholine.

6. Nanoparticules pharmaceutiquement compatibles selon l'une quelconque des revendications précédentes, dans lesquelles les nanoparticules sont associées à un ou plusieurs acides aminés.

7. Nanoparticules pharmaceutiquement compatibles selon la revendication 6, dans lesquelles les un ou plusieurs acides aminés sont sélectionnés parmi l'arginine et la glycine.

8. Nanoparticules pharmaceutiquement compatibles selon l'une quelconque des revendications 1 à 7, dans lesquelles au moins 90 % du fluorouracile sont physiquement associés à ou absorbés sur la surface des nanoparticules de silicium et/ou d'une bicouche de phospholipide.

9. Nanoparticules pharmaceutiquement compatibles selon l'une quelconque des revendications 1 à 8, pour leur utilisation comme médicament.

10. Nanoparticules pharmaceutiquement compatibles selon l'une quelconque des revendications 1 à 8, pour leur utilisation dans le traitement du carcinome basocellulaire superficiel, des kératoses actiniques, des kératoses solaires, de l'acné ou des cicatrices.

11. Nanoparticules pharmaceutiquement compatibles pour leur utilisation selon la revendication 10, dans lesquelles les cicatrices sont sélectionnées parmi une ou plusieurs parmi les cicatrices chéloïdes, les cicatrices hypertrophiques ou les cicatrices après chirurgie.

12. Nanoparticules pharmaceutiquement compatibles pour leur utilisation selon la revendication 11, dans lesquelles la cicatrice est une cicatrice hypertrophique.
